# FASCICULE DE BREVET EUROPEEN

(11) **EP 1 607 047 B1**
(45) Date de publication et mention de la délivrance du brevet: **07.05.2008**
(21) Numéro de dépôt: 05356102.3
(22) Date de dépôt: 13.06.2005
(51) Int. Cl.: A61B 17/04

(54) **Dispositif d'ancrage chirurgical**
Chirurgische Ankervorrichtung
Surgical anchoring device

(30) Priorité: 15.06.2004 FR 0406466
(43) Date de publication de la demande: 21.12.2005
(73) Titulaire: Analytic Biosurgical Solutions - ABISS, 42000 St Etienne (FR)
(72) Inventeur: Beraud, Jean-Marc, 42100 St Etienne (FR); Delorme, Emmanuel, 71100 Chalon sur Saone (FR)
(74) Mandataire: Blanchard, Eugène Gilles

(56) Documents cités:
- WO-A-20/04017845
- DE-A- 4 302 895
- GB-A- 2 337 934
- US-A- 5 549 619
- US-A- 5 893 856
- US-A1- 2001 012 941
- US-A1- 2001 037 130
- US-A1- 2003 181 946

## Description

La présente invention concerne le domaine technique des dispositifs utilisés pour le traitement des troubles de la statique pelvienne, tels que, par exemple, l'incontinence d'effort, la cystocèle, la rectocèle et/ou le prolapsus du dôme vaginal.

Dans le domaine ci-dessus et, notamment, pour le traitement de l'incontinence d'effort, le document WO 2004/017845 présente un dispositif chirurgical pour l'ancrage des tissus musculaires ou ligamentaires d'un implant prothétique qui possède une forme générale en harpon. Dans ce même domaine, il est également connu de mettre en oeuvre d'une bandelette sous urétrale présentant deux bras de suspension opposés. Un brevet US 6 387 041 a ainsi proposé de venir fixer chacun des bras de la bandelette au niveau d'un os du pubis, au moyen d'une agrafe prévue à cet effet engagée à force dans ledit os.

Toutefois, cette technique n'est pas toujours adaptée, notamment en raison de l'effort qu'il est nécessaire d'appliquer pour assurer l'insertion de l'agrafe dans l'os.

Ainsi, il a été proposé une autre procédure de mise en place de la bandelette sous urétrale, consistant, notamment, à venir insérer les bras de suspension de cette dernière dans les ligaments et les muscles trans-obturateurs avec, éventuellement, une suture desdits bras sur les ligaments.

Une telle procédure donne particulièrement satisfaction, notamment dans le cas d'une mise en place sans tension de la bandelette.

Toutefois, à l'usage, il est apparu nécessaire de pouvoir appliquer une légère tension à la bandelette, particulièrement à ses bras de suspension, de manière à contrôler au mieux la contrainte appliquée à l'urètre, afin de faire obstacle à l'incontinence d'effort, sans toutefois empêcher une vidange totale de la vessie lors d'une miction volontaire.

Or, l'immobilisation des bras de suspension, au moyen de points de suture, ne permet pas toujours d'obtenir un réglage aussi fin que souhaité de la tension appliquée à la bandelette.

Afin d'apporter une solution à ce problème du réglage optimal de la tension appliquée sur la bandelette sous urétrale et de la position de la bandelette, problème qui se trouve être commun également au traitement d'autres pathologies, telles que par exemple la cystocèle, la rectocèle ou le prolapsus du dôme vaginal, l'invention propose un dispositif pour l'ancrage dans des tissus musculaires ou ligamentaires d'un implant prothétique pourvu de bras ou de liens de suspension souples.

Selon l'invention, le dispositif d'ancrage est caractérisé en ce qu'il comprend :
- un corps allongé,
- une tête d'ancrage située à une extrémité du corps et pourvue de moyens d'ancrage dans les tissus,
- et au moins un orifice qui est aménagé dans le corps allongé pour l'accrochage d'un bras de suspension et qui est associé à des moyens anti-retour adaptés pour permettre un réglage de la longueur utile du bras et empêcher un glissement du bras dans un sens inverse à son sens d'introduction.
caractérisé en ce qu'il présente une forme générale d'hameçon en "U" ou en "J ", la tête d'ancrage (3) comprenant une branche d'ancrage (6) recourbée en "U" dont un segment (7) est lié au corps (2), tandis que l'autre segment (8) est sensiblement parallèle au corps (2), les deux segments (7, 8) étant reliées par une âme arquée (10) dont la concavité est orientée vers le corps (2) du dispositif.

La constitution du dispositif selon l'invention permet ainsi une mise en place en deux temps dans le cadre par exemple du traitement de l'incontinence d'effort au moyen d'une bandelette sous urétrale. Tout d'abord chaque dispositif selon l'invention, avec un bras de suspension introduit dans l'orifice d'accrochage, est placé dans la région ligamentaire souhaitée. Une fois ces mises en place réalisées, il est exercé une traction progressive sur les bras de suspension, la présence des moyens anti-retour de chaque dispositif d'ancrage selon l'invention permet alors, un arrêt de la traction afin de tester l'incontinence d'effort, avec un blocage simple et automatique de la bandelette sans avoir à faire de sutures. Ainsi, il peut être testé, autant que nécessaire, l'incontinence d'effort pour atteindre le niveau de tension optimal correspondant à celui qui fait obstacle à l'incontinence d'effort mais n'empêche pas une vidange complète de la vessie lors de la miction. Un tel réglage par itération successive ne serait pas aussi simple à réaliser dans le cadre d'une procédure où l'immobilisation définitive des bras est assurée par sutures.

Selon l'invention les moyens anti-retour peuvent être réalisés de toute façon appropriée.

Dans une forme préférée, mais non limitative de réalisation de l'invention, l'orifice d'accrochage traverse le corps du dispositif d'ancrage, depuis une face d'introduction vers une face de sortie du bras de suspension et les moyens anti-retour comprennent au moins une dent située au niveau de la face de sortie.

Ainsi, lorsqu'une traction est exercée sur le bras de suspension dans le sens inverse à son sens d'introduction, le bras vient s'engager sur la dent qui en bloque le mouvement de manière automatique sans autre action du chirurgien.

De manière préférée, afin de favoriser cet auto accrochage du bras de suspension, l'orifice d'accrochage présente une section décroissante depuis sa face d'introduction vers sa face de sortie.

Selon l'invention, la tête d'ancrage peut être réalisée de différentes manières, en fonction notamment de la tension qui sera appliquée au bras de suspension à y être fixé ou, encore, de la configuration de la zone tissulaire dans laquelle elle sera intégrée.

Selon une caractéristique de l'invention, la tête d'ancrage présente une forme lancéolée, telle que, par exemple, une forme de flèche en « V ».

Selon une autre caractéristique de l'invention, la tête d'ancrage présente une série de dents d'ancrage réparties de part et d'autre du corps qui donnent ainsi une forme en sapin à la tête d'ancrage.

Afin d'assurer un meilleur ancrage du dispositif dans les tissus, la face interne de la branche en « U » peut alors comprendre au moins une dent d'ancrage.

Une telle forme de réalisation du dispositif selon l'invention, telle que décrite précédemment, est particulièrement adaptée à une mise en place au moyen d'un porte-aiguille. Toutefois, selon une autre caractéristique de l'invention, afin de faciliter la mise en place du dispositif d'ancrage dans les tissus cibles, le corps du dispositif peut comprendre, à l'opposé de la tête d'ancrage, des moyens d'adaptation sur la tige d'un guide d'introduction.

De tels moyens d'adaptation peuvent être réalisés de toute façon appropriée telle que, par exemple, en adoptant, pour l'extrémité opposée à la tête d'ancrage du corps du dispositif, une forme à section polygonale destinée à venir s'engager dans un logement complémentaire du guide d'introduction.

Il pourra également être envisagé de réaliser ces moyens d'adaptation sous la forme d'aspérités, permettant la prise en charge du dispositif d'ancrage selon l'invention par un porte-aiguille par exemple. De telles aspérités pourront alors être réalisées, soit à l'extrémité du corps du dispositif d'ancrage opposé à la tête d'ancrage, soit, au contraire, au milieu du corps allongé, entre, par exemple, la tête d'ancrage et l'orifice d'accrochage d'un bras de suspension.

Selon l'invention la tête d'ancrage est susceptible de présenter différentes formes. Ainsi, il pourrait être envisagé que la tête d'ancrage comprenne plus d'une branche d'ancrage recourbée en « U » et, par exemple, trois branches qui sont, par exemple, disposées à 120° les unes des autres et qui confèrent ainsi une forme générale de grappin au dispositif d'ancrage selon l'invention.

Selon l'invention, le dispositif d'ancrage peut être réalisé en tout matériau biocompatible, naturel ou non, présentant une dureté suffisante pour assurer sa fonction d'ancrage dans les tissus.

Dans une forme de réalisation préférée, il sera utilisé un matériau biocompatible à résorption lente tel que choisi dans les polymères bio résorbables dont les plus utilisés en chirurgie sont des polyglycolides et polylactides. Bien entendu le dispositif selon l'invention pourrait également être réalisé en métal.

Diverses autres caractéristiques de l'invention ressortent de la description ci-dessous effectuée en référence aux dessins annexés qui illustrent différentes formes de réalisation d'un dispositif d'ancrage conforme à l'invention.
La **fig. 1** est une perspective d'une forme de réalisation en hameçon d'un dispositif d'ancrage selon l'invention.
La **fig. 2** est une coupe longitudinale du dispositif d'ancrage selon la **fig. 1**.
La **fig. 3** est une perspective d'une variante de réalisation en forme de grappin d'un dispositif d'ancrage selon l'invention.
La **fig. 4** est une élévation d'une variante de réalisation en forme de sapin d'un dispositif d'ancrage selon l'invention.
La **fig. 5** est une élévation d'une variante de réalisation en forme de flèche d'un dispositif d'ancrage selon l'invention.

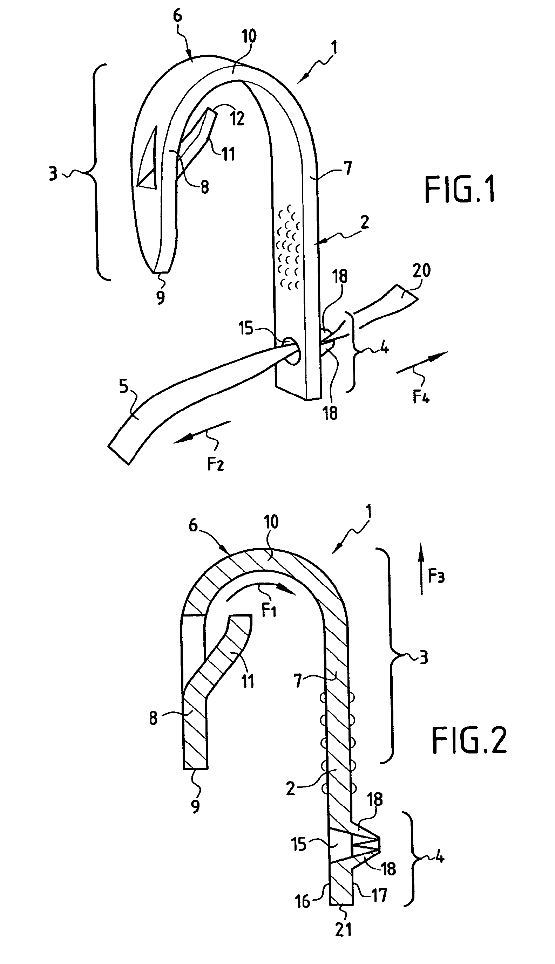

Un dispositif d'ancrage selon l'invention, tel qu'illustré aux **fig. 1** et **2** et désigné dans son ensemble par la référence **1**, comprend un corps allongé **2** pourvu, à une extrémité, d'une tête **3** d'ancrage dans des régions tissulaires, telles que des régions musculaires ou ligamentaires, et, à l'opposé de la tête **3**, de moyens **4** d'accrochage pour un bras **5** de suspension d'une bandelette sous urétrale **5**.

Selon l'exemple illustré, la tête d'accrochage **3** présente une branche en « U » **6**, dont un segment **7** est relié au corps, tandis que l'autre segment **8** est sensiblement parallèle au premier segment **7** et au corps **2**. Le deuxième segment **8** présente alors une extrémité pointue **9** et forme ainsi un moyen d'ancrage dans des tissus. Le premier segment **7** est relié à au second segment **8** par une âme arquée **10** dont la concavité est orientée vers le corps du dispositif de sorte dispositif d'ancrage selon cet exemple de réalisation présente une forme générale d'hameçon.

Selon l'exemple illustré, la tête d'ancrage **3** comprend, en outre, une dent d'ancrage **11**, aménagée sur la face interne de la branche en U **6**, en étant, selon l'exemple illustré, sensiblement placée dans la région de liaison entre le second segment **8** et l'âme **10**, la pointe **12** de la dent **11** étant orientée vers l'âme de liaison **10** de manière à empêcher tout rotation ou glissement intempestif selon la flèche **F₁** du dispositif **1** dans les tissus dans lesquels il est accroché.

A l'opposé de la tête **3**, le corps **2** présente les moyens **4** d'accrochage anti-retour du bras de suspension **5**. Selon l'exemple illustré, ces moyens d'accrochage anti-retour comprennent un orifice **15** traversant le corps allongé **2**, depuis une face **15** dite d'introduction jusqu'à une face **16** dite de sortie du bras de suspension **5**. Selon l'exemple illustré la face d'introduction **16** est située en regard du second segment **8** et la face de sortie **17** est orientée vers l'extérieur mais une disposition inverse pourrait tout aussi bien être envisagée.

Selon l'exemple illustré, l'orifice d'accrochage **15** présente une section décroissante depuis la face d'introduction **16** vers la face de sortie **17**.

L'orifice **15** se trouve, en outre, complété par des moyens anti-retour, comprenant, selon l'exemple illustré, deux dents **18** situées en regard l'une de l'autre au niveau de la face de sortie, pour un accrochage ou un arrêt automatique du bras de suspension **5** lors d'une traction dans le sens de la flèche **F₂** à l'inverse du sens d'introduction. Il est à noter qu'afin de favoriser cet effet anti-retour le diamètre de l'orifice **15** est choisi pour être inférieur à la largeur du bras de suspension **5**.

Selon l'exemple illustré, le dispositif d'ancrage **1** comprend, en outre, au niveau du corps **2**, entre la tête d'ancrage **3** et les moyens **4** d'accrochage du bras de suspension, deux séries d'aspérités **16** aménagées sur ses deux faces, de manière à permettre une prise en charge du dispositif d'ancrage par un porte-aiguille par exemple.

Un tel dispositif d'ancrage **1** conforme à l'invention est mis en oeuvre de la manière suivante.

Tout d'abord, l'extrémité d'un bras de suspension 5 est engagée dans l'orifice **15** de manière à laisser dépasser du coté de la face de sortie **17** une longueur **20** de bras suffisante pour être prise à deux doigts ou au moyen d'une pince.

Le dispositif **1** est ensuite engagé dans les tissus, de manière à orienter la convexité de la tête d'ancrage **3** dans le sens **F₃** d'introduction pour présenter une moindre résistance à la pénétration dans les tissus.

Lorsque la position optimale du dispositif **1** est atteinte, le chirurgien arrête la poussée exercée sur le dispositif au niveau de son corps et exerce ensuite une traction, dans le sens de la flèche **F₄**, sur la partie **20** du bras de suspension **5** pour régler la longueur utile du bras et la tension de la bandelette solidaire dudit bras. On entend ici par longueur utile la longueur de la partie du bras qui contribue effectivement à la suspension et reprend les efforts par opposition à la partie **20** bras qui pourrait être qualifiée de partie libre.

Il est à noter qu'une traction en sens inverse **F₂** assure un accrochage automatique du bras de suspension sur les deux dents **18** qui présentent donc une fonction anti-retour, de sorte que, par l'entremise du dispositif **1**, le bras de suspension **5** se trouve littéralement ancré dans les tissus au niveau duquel le dispositif **1** est inséré. Ainsi, par la mise en oeuvre de ce dispositif d'ancrage **1**, le chirurgien peut très rapidement positionner les bras de suspension dans la zone d'ancrage privilégiée et ensuite assurer le réglage de la longueur utile et de la tension appliquée sur les bras de suspension. Ce réglage devra être effectué par petites touches, afin de ne pas dépasser la tension maximum au-delà de laquelle la miction ou la vidange totale de la vessie n'est plus possible. Cependant, dans la mesure où la tension et la position du bras est réglable par simple traction, il est possible de procéder par itération et ainsi d'atteindre le niveau de tension optimal après avoir effectué une série de tests. Une fois le réglage de la tension effectuée le chirurgien coupera la portion excédentaire de la partie **20** du bras de suspension en veillant à ne pas couper trop près du dispositif d'ancrage afin de préserver la fiabilité de l'accrochage du bras de suspension sur le dispositif d'ancrage.

Il convient en outre de noter que le dispositif d'ancrage selon l'invention peut être utilisé soit dans le cadre d'une chirurgie traditionnelle soit dans le cadre d'une chirurgie par célioscopie avec un ancillaire spécifique ou un porte aiguille.

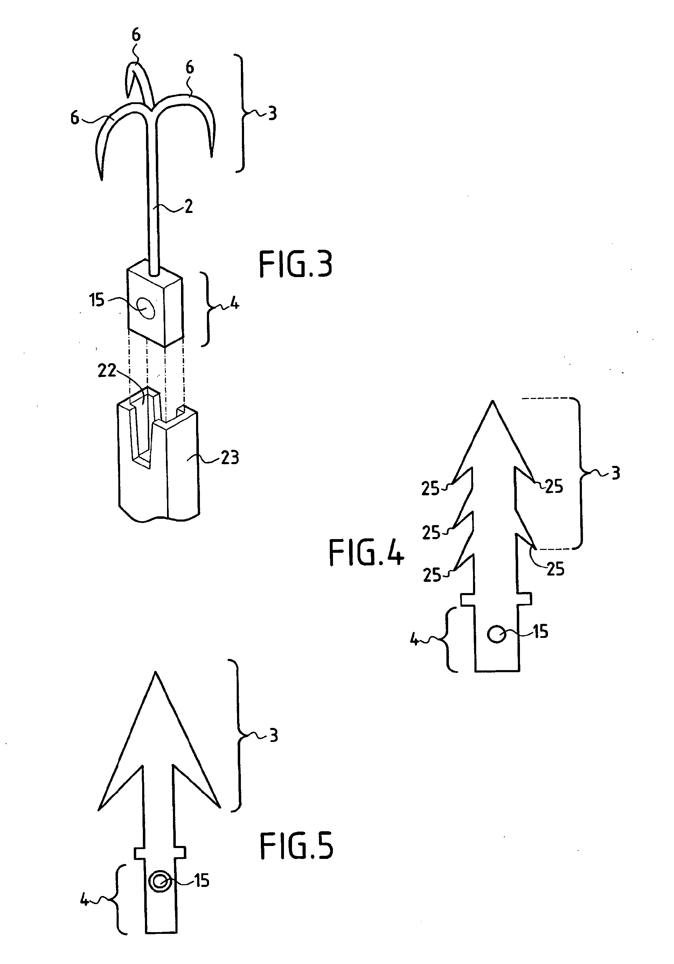

Selon l'invention, la tête **3** du dispositif d'ancrage **1** pourrait présenter plus d'une branche recourbée **6**, telle qu'illustrée aux **fig. 1** et **2**. Ainsi, la **fig. 3** illustre une autre forme de réalisation d'une tête d'ancrage **3** à trois branches en U **6**, conférant une forme de grappin au dispositif d'ancrage **1**.

Il est à noter que, selon l'exemple illustré, le corps **2** du dispositif d'ancrage **1** présente, à l'opposé de la tête d'ancrage, une section droite transversale sensiblement rectangulaire, de sorte que l'extrémité **21** du corps **2** est susceptible d'être engagée au niveau d'un évidemment **22** de forme complémentaire aménagé dans un ancillaire, tel que, par exemple, un guide d'introduction rigide ou semi-rigide **23** dont seule une extrémité est illustrée à la **fig. 3**. La forme de l'extrémité **21** corps **2** constitue ainsi des moyens d'adaptation du dispositif **1** sur le guide **23**. Bien entendu, selon l'invention, il pourrait être envisagé un autre mode de réalisation des moyens d'adaptation du dispositif sur un ancillaire.

Selon l'invention, la tête d'ancrage pourrait être réalisée de diverses autres manières que celles illustrées fig. 1 et 3.

Ainsi, la **fig. 4** illustre encore une autre forme de réalisation selon laquelle la tête d'ancrage **3** présente une série de dents **25**, réparties de part et d'autre du corps **2**, conférant ainsi une forme de sapin au dispositif **1**.

De même, la **fig. 5** illustre une autre forme de réalisation selon laquelle la tête d'ancrage **3** présente une forme de flèche en « V ».

Bien entendu, différentes autres modifications peuvent être apportées à l'invention sans sortir de son cadre.

## Revendications

1. Dispositif chirurgical pour l'ancrage dans des tissus musculaires ou ligamentaires d'un implant prothétique, pourvu d'au moins un bras ou lien (**5**) de suspension souple, comprenant :
• un corps allongé (**2**),
• une tête d'ancrage (**3**) située à une extrémité du corps (**2**) et pourvue de moyens (**6**, **8**, **11**, **25**) d'ancrage dans les tissus,
• et au moins un orifice (**15**) qui est aménagé dans le corps allongé (**2**) pour l'accrochage du bras de suspension (**5**) et qui est associé à des moyens anti-retour (**18**) adaptés pour permettre un réglage de la longueur utile du bras et empêcher un glissement du bras (**5**) dans un sens inverse à son sens d'introduction,
**caractérisé en ce qu'**il présente une forme générale d'hameçon en « U » ou en « J », la tête d'ancrage **(3)** comprenant une branche d'ancrage **(6)** recourbée en « U » dont un segment **(7)** est lié au corps **(2),** tandis que l'autre segment **(8)** est sensiblement parallèle au corps **(2),** les deux segments (**7**, **8**) étant reliées par une âme arquée **(10)** dont la concavité est orientée vers le corps (**2**) du dispositif.

2. Dispositif d'ancrage selon la revendication 1, **caractérisé en ce qu'**il comprend au moins une dent d'ancrage **(11)** aménagée sur la face interne de la branche en « U » **(6).**

3. Dispositif d'ancrage selon la revendication 1 ou 2, **caractérisé en ce que** l'orifice d'accrochage (**15**) traverse le corps du dispositif depuis une face d'introduction (**16**) vers une face de sortie (**17**) et **en ce que** les moyens anti-retour comprennent au moins une dent (**18**) située au niveau de la face de sortie pour un accrochage du bras de suspension (**5**).

4. Dispositif d'ancrage selon la revendication 3, **caractérisé en ce que** l'orifice d'accrochage (**15**) présente une section décroissante, depuis la face d'introduction (**16**) vers la face de sortie (**17**).

5. Dispositif d'ancrage selon l'une des revendications 1 à 4, **caractérisé en ce qu'**il est réalisé dans un matériau biocompatible à résorption lente.

6. Dispositif d'ancrage selon l'une des revendications 1 à 5, **caractérisé en ce qu'**il comprend, à l'opposé de la tête d'ancrage, des moyens (**21**) d'adaptation sur la tige d'un guide d'introduction (**23**).

## Claims

1. A surgical device for anchoring a prosthetic implant in muscle or ligament tissues, provided with at least one flexible suspension arm or linkage (5), comprising:
• an elongated body (2),
• an anchoring head (3) located at one end of the body (2) and provided with means (6, 8, 11, 25) for anchoring in tissues,
• and at least one orifice (15) which is made in the elongated body (2) for suspending the suspension arm (5) and which is associated with suitable anti-return means (18) for allowing the useful length of the arm to be adjusted and preventing sliding of the arm (5) in an opposite direction to its direction of introduction,
**characterized in that** it has a general "U" or "J" hook shape, the anchoring head (3) comprising a U-curved anchoring branch (6), a segment (7) of which is bound to the body (2), whereas the other segment (8) is substantially parallel to the body (2), both segments (7, 8) being connected by an arched web (10), the concavity of which is oriented towards the body (2) of the device.

2. The anchoring device according to claim 1, **characterized in that** it comprises at least one anchoring tooth (11) made on the internal face of the "U" branch (6).

3. The anchoring device according to claim 1 or 2, **characterized in that** the suspension orifice (15) passes through the body of the device from an introduction face (16) to an output face (17) and **in that** the anti-return means comprise at least one tooth (18) located at the output face for suspending the suspension arm (5).

4. The anchoring device according to claim 3, **characterized in that** the suspension orifice (15) has a decreasing section, from the introduction face (16) to the output face (17).

5. The anchoring device according to any of claims 1 to 4, **characterized in that** it is made in a slow resorption biocompatible material.

6. The anchoring device according to any of claims 1 to 5, **characterized in that** it comprises, opposite to the anchoring head, adaptation means (21) on the rod of an introduction guide (23).

## Patentansprüche

1. Chirurgische Vorrichtung für die Verankerung eines Protheseimplantats, das mit mindestens einem flexiblen Aufhängungsarm oder -band versehen ist, in Muskelgeweben oder -bändern, umfassend:
- einen länglichen Körper (2),
- einen Verankerungskopf (3), der sich an einem Ende des Körpers (2) befindet und mit Mitteln (6, 8, 11, 25) zur Verankerung in den Geweben versehen ist,
- und mindestens eine Öffnung (15), die in dem länglichen Körper (2) für die Befestigung des Aufhängungsarms (5) vorgesehen und mit Rückzugverhinderungsmitteln (18) verbunden ist, die dazu ausgeführt sind, eine Einstellung der Nutzlänge des Arms zu ermöglichen und ein Gleiten des Arms (5) in eine zu seiner Einführungsrichtung umgekehrte Richtung zu verhindern,
**dadurch gekennzeichnet, daß** sie eine allgemeine Angelhakenform in Form eines "U" oder "J" aufweist, wobei der Verankerungskopf (3) einen zu einem "U" gekrümmten Verankerungsabschnitt (6) umfaßt, bei dem ein Segment (7) mit dem Körper (2) verbunden ist, während das andere Segment (8) im wesentlichen zum Körper (2) parallel ist, wobei die beiden Segmente (7, 8) durch einen gewölbten Mittelteil (10) verbunden sind, dessen Konkavität zum Körper (2) der Vorrichtung gerichtet ist.

2. Verankerungsvorrichtung nach Anspruch 1, **dadurch gekennzeichnet, daß** sie mindestens einen Verankerungszahn (11) umfaßt, der auf der Innenseite des U-förmigen Abschnitts (6) angeordnet ist.

3. Verankerungsvorrichtung nach Anspruch 1 oder 2, **dadurch gekennzeichnet, daß** die Befestigungsöffnung (15) den Körper der Vorrichtung von einer Einführungsseite (16) zu einer Ausgangsseite (17) durchquert, und daß die Rückzugsverhinderungsmittel mindestens einen Zahn (18) umfassen, der sich im Bereich der Ausgangsseite befindet, um den Aufhängungsarm (5) zu befestigen.

4. Verankerungsvorrichtung nach Anspruch 3, **dadurch gekennzeichnet, daß** die Befestigungsöffnung (15) einen von der Einführungsseite (16) zur Ausgangsseite (17) abnehmenden Querschnitt aufweist.

5. Verankerungsvorrichtung nach einem der Ansprüche 1 bis 4, **dadurch gekennzeichnet, daß** sie aus einem biologisch verträglichen Material mit langsamer Resorption besteht.

6. Verankerungsvorrichtung nach einem der Ansprüche 1 bis 5, **dadurch gekennzeichnet, daß** sie gegenüber dem Verankerungskopf Anpassungsmittel (21) auf dem Schaft einer Einführungsführung (23) umfaßt.
